# EUROPEAN PATENT SPECIFICATION

(11) **EP 3 592 395 B1**
(45) Date of publication and mention of the grant of the patent: **04.06.2025**
(21) Application number: 18712384.9
(22) Date of filing: 07.03.2018
(51) Int. Cl.: A61L 15/32, A61L 15/60, A61L 15/62

(54) **DEVICE FOR DELIVERY OF POWDERED HEMOSTATIC AGENTS**
VORRICHTUNG ZUR ABGABE VON PULVERFÖRMIGEN BLUTSTILLERN
DISPOSITIF D'ADMINISTRATION D'AGENTS HÉMOSTATIQUES EN POUDRE

(30) Priority: 08.03.2017 US 201762468797 P
(43) Date of publication of application: 15.01.2020
(73) Proprietor: Baxter International Inc., Deerfield, IL 60015 (US); Baxter Healthcare SA, 8152 Glattpark (Opfikon) (CH)
(72) Inventor: HOLSCHER, Russel L., Deerfield, IL 60015 (US); SANDERS, Paul Jefferey, Greendale, Wisconsin 53129 (US); FULGHUM, Timothy Michael, Lakemoor, Illinois 60073 (US); BARRY, John Joseph Anthony, Northbrook, Illinois 60062 (US)
(74) Representative: SONN Patentanwälte GmbH & Co KG
(86) International application number: PCT/US2018/021313
(87) International publication number: WO 2018/165275

(56) References cited:
- US-A1- 2007 276 345
- US-A1- 2013 084 323
- US-A1- 2013 095 165

## Description

### FIELD OF THE INVENTION

The disclosure relates generally to multilayered hemostatic devices. More particularly, the disclosure relates to multilayered hemostatic devices including a powdered hemostat and a rapidly dissolving/degrading film.

### BACKGROUND

Hemostatic agents and sealants are currently used as an aid to stop bleeding, including hemorrhaging during surgery. Hemostatic agents are typically presented in the form of solid fabrics, powders or granules, or as liquids. Flowable hemostatic agents provide for good contact with irregular surfaces which are typical of wounds so that good hemostasis can be achieved.

US 2007/276345 A1 discloses a device capable of providing a hemostatic effect on a bleeding wound, said device comprising:
- a gauze substrate;
- a clay material disposed on said gauze substrate;
- a polyol disposed on said gauze substrate to bind said clay material; and
- further comprising a release agent disposed on said clay material;
wherein when treating a bleeding wound, application of said device causes at least a portion of said clay material to come into contact with blood.

However, the flowable nature of particulate hemostatic agents also renders them relatively difficult to handle in use. Current powdered hemostats are conventionally applied with plastic bellow type applicators. Controlled and precise application of powders with this type of delivery system can be challenging, resulting, for example, in the hemostatic agent being blown away from the wound site such that a significant portion of the hemostatic agent can be wasted.

### SUMMARY

The scope of the invention is defined by the claims. Any references in the description to methods of treatment refer to the products of the present invention for use in a method for treatment.

A multilayered hemostatic device is provided, the device comprising:
- a wound-contacting first layer comprising carboxymethyl cellulose, the first layer being soluble under physiological conditions, such that the first layer will dissolve when in contact with a fluid from a wound;
- a second layer comprising a flowable powdered hemostatic agent provided on the first layer, the hemostatic agent comprising one of fibrinogen, thrombin, fibrin monomers, or the combination of thrombin and gelatin; and
- a third layer comprising at least one of gauze, oxidized cellulose, or collagen provided on the second layer,
wherein the first layer and the third layer encapsulate the second layer, wherein the first layer and the third layer have perimeter edges and the first layer and third layer are sealed along the perimeter edges; and wherein the rate of dissolution of the first layer under physiological conditions is greater than the rate of degradation of the third layer under physiological conditions.

### BRIEF DESCRIPTION OF THE DRAWINGS

For further facilitating the understanding of the present invention, two drawing figures are appended hereto.

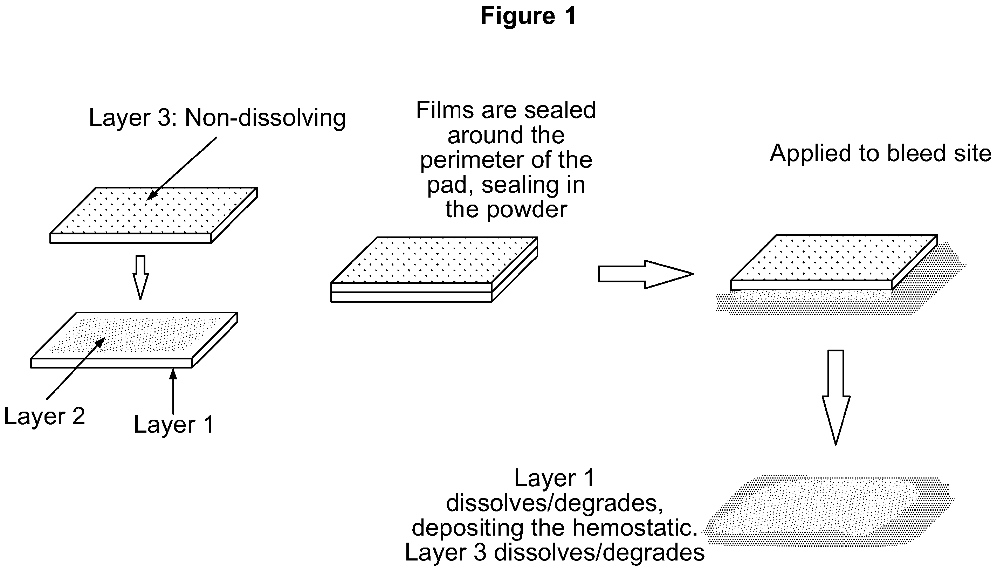

Figure 1 shows a rendering of a multilayer device comprising a first soluble/degradable layer, a second powdered hemostatic agent layer, and a third soluble/degradable layer, and also shows after application of the device to a wound such that the first layer is in contact with the wound, the dissolution/degradation of the first layer to permit direct contact of the hemostatic agent with the wound and the subsequent dissolution/degradation of the third layer.

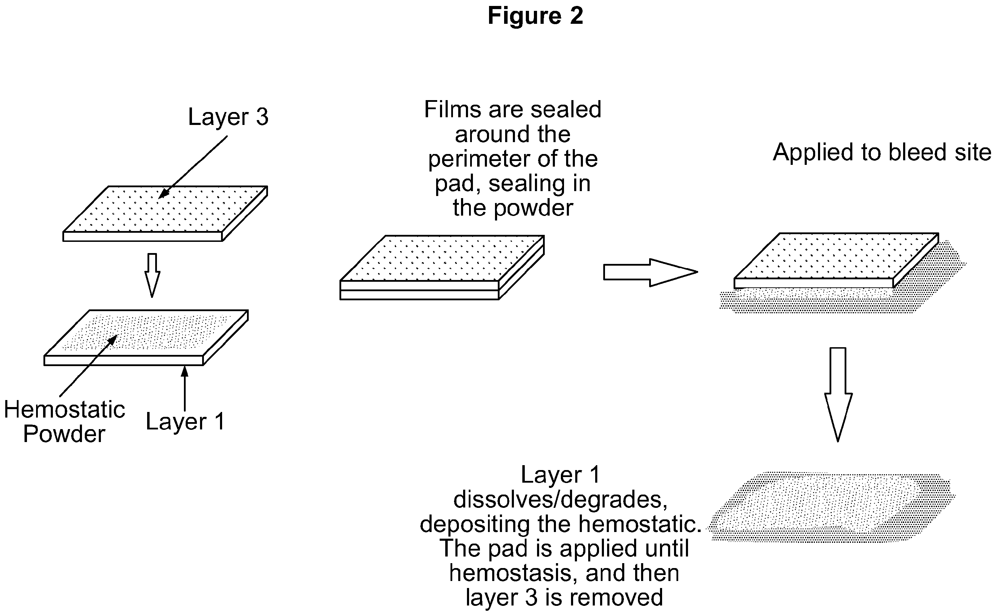

Figure 2 shows a rendering of a multilayer device comprising a first soluble/degradable layer, a second powdered hemostatic agent layer, and a third non- soluble and non-degradable layer, and also shows after application of the device to a wound such that the first layer is in contact with the wound, the dissolution/degradation of the first layer to permit direct contact of the hemostatic agent with the wound and the subsequent removal of the third layer.

### DETAILED DESCRIPTION

Advantageously, the multilayered hemostatic device of the disclosure allows for the precise, controlled delivery and application of flowable, powdered, hemostatic agents to a wound site in a manner which maximizes the utilization of the powdered hemostatic agent at the surgical site, without waste resulting from imprecise delivery or blowing away of the hemostatic agent from the site of delivery.

As used herein, "powdered hemostatic agent" refers to a flowable, solid particulate material. "Powdered" and "particulate" may be used interchangeably herein.

In embodiments, the powdered hemostatic agent is provided on the first layer in a repeating pattern. In embodiments, the perimeter edges of the first layer and third layer are sealed.

The first layer is soluble under physiological conditions, such that the first layer will dissolve when in contact with a fluid from a wound. Physiological conditions encompass aqueous-based milieu having a temperature in a range of about 20 to about 40 °C and a pH of about 6 to about 8, and an atmospheric pressure of about 0.8 atm to about 1.2 atm and corresponding atmospheric oxygen concentrations. In embodiments, the first layer is degradable under physiological conditions, such that the first layer will degrade when in contact with a fluid from a wound. In embodiments, the third layer is degradable under physiological conditions, such that the third layer will degrade when in contact with a fluid from a wound. In embodiments, the third layer forms a gel under physiological conditions. In embodiments, the third layer is not-soluble and/or not-degradable under physiological conditions, such that the third layer will not dissolve or degrade when in contact with a fluid from a wound and, after application of the device to the wound and dissolution and/or degradation of the first layer, the second layer is disposed between a wound and the third layer when the hemostatic device is applied to the wound.

### FIRST LAYER

The device of the disclosure comprises a first layer which is the wound-contacting layer. The first layer is soluble and/or degradable under physiological conditions, such that the first layer will dissolve and/or degrade when in contact with a fluid from a wound (e.g., blood and/or wound exudate). In embodiments, the first layer is rapidly soluble and/or degradable under physiological conditions and will dissolve and/or degrade after less than about 90 seconds of contact with a fluid from a wound, after less than about 60 seconds of contact with a fluid from a wound, or after less than about 30 seconds of contact with a fluid from a wound.

The first layer comprises carboxymethyl cellulose and may be provided as a knitted, non-woven fabric, as a woven fabric, or as a soluble/degradable film. Other materials for use in addition to carboxymethyl cellulose include, but are not limited to, polyvinyl alcohols and modified polyvinyl alcohols, polyacrylates, water-soluble acrylate copolymers, polyvinyl pyrrolidone, pullulan, gelatin, hydroxylpropylmethyl cellulose (HPMC), polyethylene oxide, polyethylene glycols, low viscosity grade hydroxypropylcellulose, polysaccharides, water-soluble natural polymers including, but not limited to, guar gum, xanthan gum, locust bean gum, carrageenan, and starch, modified starches including, but not limited to, ethoxylated starch and hydroxypropylated starch, copolymers of the foregoing and combinations of any of the foregoing.

Soluble/degradable films according to the present disclosure may include other optional additive ingredients including, but not limited to, plasticizers, surfactants, film formers, and other functional ingredients, for example in amounts suitable for their intended purpose.

Suitable plasticizers may include glycerol, diglycerol, propylene glycol, ethylene glycol, diethylene glycol, triethylene glycol, tetraethylene glycol, polyethylene glycols up to MW 400, phthalate derivatives, including but not limited to, dimethyl phthalate, diethyl phthalate, and dibutyl phthalate, citrate derivatives, including but not limited to tributyl citrate, triethyl citrate, acetyl citrate, triacetin citrate, and castor oil.

The first layer is soluble under physiological conditions, such that the first layer will dissolve when in contact with a fluid from a wound. In embodiments, the first layer is degradable under physiological conditions, such that the first layer will degrade when in contact with a fluid from a wound.

### SECOND LAYER

The device of the disclosure comprises a second layer comprising a powdered hemostatic agent provided on the first layer. The second, powdered hemostatic agent layer may be a homogenous layer or a heterogeneous layer. In embodiments, the powdered hemostatic agent is provided on the first layer in a repeating pattern, for example, the powdered hemostatic agent can be deposited on the first layer as a pattern of discrete dots, as pattern of discrete columns or rows, or as a repeating pattern of other geometric shapes.

As used herein, "hemostatic agent" refers to a material that exerts a passive or active mode of hemostatic action upon a bleeding wound.

The powdered hemostatic agent is selected from the group consisting of fibrinogen, thrombin, fibrin monomers, gelatin, and mixtures of the foregoing. The powdered hemostatic agent may be a lyophilized hemostatic agent, such as lyophilized thrombin or lyophilized fibrinogen. In embodiments, the powdered hemostatic agent is selected from the group consisting of fibrinogen, thrombin, fibrin monomers, the combination of thrombin and gelatin, and mixtures of the foregoing. In embodiments, the powdered hemostatic agent comprises fibrinogen. In embodiments, the powdered hemostatic agent is fibrinogen. In embodiments, the powdered hemostatic agent comprises fibrin monomers. In embodiments, the powdered hemostatic agent is fibrin monomers. In embodiments, the powdered hemostatic agent comprises thrombin. In embodiments, the powdered hemostatic agent comprises thrombin and gelatin. In embodiments, the powdered hemostatic agent is thrombin. In embodiments, the powdered hemostatic agent is the combination of thrombin and gelatin.

As used herein, "fibrin monomer" includes any form of fibrin, e.g., fibrin I, fibrin II, or des BB fibrin, wherein the fibrin is in monomeric form or oligomeric form that can be solubilized and can be converted to fibrin polymer. Fibrin oligomers are capable of being solubilized under physiological conditions whereas fibrin polymer is not soluble under physiological conditions.

Other powdered/particulate hemostatic agents for use in addition to fibrinogen, thrombin, fibrin monomer, and gelatin include, but are not limited to, oxidized cellulose, chitosan, alginate, polysaccharides, ethyl cellulose beads and beads of other oxidized celluloses, maltodextrin, clotting factor concentrates, recombinant Factor Vila, alphanate FVIII concentrate, bioclate FVm concentrate, monoclate-P FVHI concentrate, haemate P FVIII, von Willebrand factor concentrate, helixate FVII concentrate, kogenate FVII concentrate, recombinate FVIII concentrate, mononine FIX concentrate, fibrogammin P FXIII concentrate, and combinations of the foregoing.

### THIRD LAYER

The device of the disclosure comprises a third layer provided on the second layer, wherein the first layer and the third layer encapsulate the second layer. In embodiments, the third layer may be soluble and/or degradable under physiological conditions, such that the third layer will dissolve and/or degrade when in contact with a fluid from a wound. In embodiments, the third layer may be non-soluble and non-degradable under physiological conditions, such that the third layer will not dissolve or degrade when in contact with a fluid from a wound, even when the contact between the wound and the third layer is for an extended period of time, for example, a period of time greater than 5 minutes, 15 minutes, or even one hour. In embodiments, the third layer may form a gel under physiological conditions.

The third layer comprises a material selected from the group consisting of gauze, oxidized cellulose, collagen, and mixtures of the foregoing. In embodiments, the third layer comprises gauze. In embodiments, the third layer is gauze. In embodiments, the third layer comprises oxidized cellulose. In embodiments, the third layer is oxidized cellulose. In embodiments, the third layer comprises collagen. In embodiments, the third layer is collagen. Optionally, the oxidized cellulose is oxidized regenerated cellulose.

Suitable other materials for use in addition to gauze, oxidized cellulose, and collagen include, but are not limited to, silicone, keratin, gelatin, chitin, chitosan, polyvinyl pyrrolidone, alginate, cross-linked polyvinyl alcohol, and combinations of the foregoing.

The third layer may also optionally include a pH modifier. Without intending to be bound by theory, it is believed that when the second layer comprises thrombin and the third layer comprises oxidized cellulose or oxidized regenerated cellulose, the acidic pH of the third layer may result in precipitation of fibrinogen from the wound before it has a chance to react with the thrombin to form a clot. Accordingly, without intending to be bound by theory, a pH modifier may be included in the third layer to provide the third layer with a neutral pH to avoid the precipitation of fibrinogen. Suitable pH modifiers include, but are not limited to, soda ash, sodium silicate, lime, sodium phosphate, and buffer compositions, for example, acetate buffers, citrate buffers, phosphate-buffered saline. pH modifiers can be provided in particulate form or can be coated on the third layer, for example, as a solution.

The device of the disclosure may have any suitable configuration, including but not limited to, triangular, quadrilateral, such as square or rectangular, circular, oblong, pentagon, hexagon, and octagon, for example. In embodiments, at least the first layer and the third layer have a similar configuration such that the first layer and third layer have substantially parallel perimeter edges to facilitate encapsulation and sealing of the second layer between the first and third layers.

The perimeter edges of the first layer and the third layer are sealed. The perimeter edges of the first layer and third layer may be sealed according to any suitable process known in the art, including but not limited to, solvent sealing and heat sealing.

As shown in Figure 1 and Figure 2, the device of the disclosure may be disposed on a wound to provide a precise delivery and application of powdered hemostatic agents. Advantageously, after the device is applied such that the first layer is in contact with the wound, the first wound-facing layer readily dissolves and/or degrades such that the hemostatic agent of the second layer is subsequently disposed between, and in contact with, the wound and the third layer. The third layer may be selected such that it will not dissolve or degrade until hemostasis of the wound has occurred or such that it will not dissolve or degrade at all and will remain in place until hemostasis of the wound has occurred and the third layer is removed.

The rate of dissolution of the first layer under physiological conditions is greater than the rate of dissolution of the third layer under physiological conditions. Further, in embodiments, the third layer is not-soluble and/or not-degradable under physiological conditions and the second layer is disposed between a wound and the third layer after the hemostatic device is applied to the wound.

In embodiments, when the hemostatic agent comprises fibrinogen, the third layer comprises collagen.

The combinations provided in Table 1, below, are each particularly contemplated embodiments. In each of the combinations provided in Table 1, it is specifically contemplated that the first layer, second layer, and third layer may comprise the listed materials or may consist of the listed materials.

| Table 1 | First Layer | Second Layer | Third Layer |
|---|---|---|---|
| 1 | Carboxymethyl cellulose | Thrombin and gelatin | Oxidized cellulose |
| 2 | Carboxymethyl cellulose | Thrombin and gelatin | Gauze |
| 3 | Carboxymethyl cellulose | Thrombin and gelatin | Collagen |
| 4 | Carboxymethyl cellulose | Fibrinogen | Oxidized regenerated cellulose |
| 5 | Carboxymethyl cellulose | Thrombin | Oxidized regenerated cellulose |
| 6 | Carboxymethyl cellulose | Fibrin monomers | Oxidized regenerated cellulose |

## Claims

1. A multilayered hemostatic device comprising: A wound-contacting first layer comprising carboxymethyl cellulose, the first layer being soluble under physiological conditions, such that the first layer will dissolve when in contact with a fluid from a wound;
a second layer comprising a flowable powdered hemostatic agent provided on the first layer, the hemostatic agent comprising one of fibrinogen, thrombin, fibrin monomers, or the combination of thrombin and gelatin; and
a third layer comprising at least one of gauze, oxidized cellulose, or collagen provided on the second layer, wherein the first layer and the third layer encapsulate the second layer,
wherein the first layer and the third layer have perimeter edges and the first layer and third layer are sealed along the perimeter edges; and wherein the rate of dissolution of the first layer under physiological conditions is greater than the rate of degradation of the third layer under physiological conditions.

2. The multilayered hemostatic device of claim 1, wherein the powdered hemostatic agent is a solid particulate.

3. The multilayered hemostatic device of claim 1 or claim 2, wherein the powdered hemostatic agent is provided on the first layer in a repeating pattern.

4. The multilayered hemostatic device of claim 1, wherein the perimeter edges of the first layer and the third layer are heat sealed.

5. The multilayered hemostatic device of any one of the preceding claims, wherein the third layer forms a gel under physiological conditions.

6. The multilayered hemostatic device of any one of the preceding claims, wherein the third layer is not-soluble and/or not-degradable under physiological conditions and the second layer is disposed between a wound and the third layer when the hemostatic device is applied to the wound.

7. The multilayered hemostatic device of any one of the preceding claims, wherein the oxidized cellulose is oxidized regenerated cellulose.

8. The multilayered hemostatic device of any one of claims 1 to 7, wherein the third layer comprises oxidized regenerated cellulose, and the hemostatic agent comprises fibrinogen, preferably wherein the hemostatic agent is fibrinogen.

9. The multilayered hemostatic device of any one of claims 1 to 7, wherein the third layer comprises oxidized regenerated cellulose, and the hemostatic agent comprises thrombin, preferably wherein the hemostatic agent is thrombin.

## Patentansprüche

1. Mehrschichtige hämostatische Vorrichtung, umfassend:
Eine wundberührende erste Schicht, die Carboxymethylcellulose umfasst, wobei die erste Schicht unter physiologischen Bedingungen löslich ist, sodass sich die erste Schicht auflöst, wenn sie mit einer Flüssigkeit aus einer Wunde in Kontakt kommt;
eine zweite Schicht, die ein fließfähiges pulverförmiges hämostatisches Mittel umfasst, das auf der ersten Schicht bereitgestellt ist, wobei das hämostatische Mittel eines von Fibrinogen, Thrombin, Fibrinmonomeren oder die Kombination von Thrombin und Gelatine umfasst; und
eine dritte Schicht, die mindestens eines von Gaze, oxidierter Cellulose oder Kollagen umfasst, das auf der zweiten Schicht bereitgestellt ist, wobei die erste Schicht und die dritte Schicht die zweite Schicht einkapseln,
wobei die erste Schicht und die dritte Schicht Umfangsränder aufweisen und die erste Schicht und die dritte Schicht entlang der Umfangsränder versiegelt sind; und wobei die Auflösungsrate der ersten Schicht unter physiologischen Bedingungen größer als die Abbaurate der dritten Schicht unter physiologischen Bedingungen ist.

2. Mehrschichtige hämostatische Vorrichtung gemäß Anspruch 1, wobei das pulverförmige hämostatische Mittel feste Partikel sind.

3. Mehrschichtige hämostatische Vorrichtung gemäß Anspruch 1 oder Anspruch 2, wobei das pulverförmige hämostatische Mittel auf der ersten Schicht in einem sich wiederholenden Muster bereitgestellt ist.

4. Mehrschichtige hämostatische Vorrichtung gemäß Anspruch 1, wobei die Umfangsränder der ersten Schicht und der dritten Schicht thermisch versiegelt sind.

5. Mehrschichtige hämostatische Vorrichtung gemäß einem der vorhergehenden Ansprüche, wobei die dritte Schicht unter physiologischen Bedingungen ein Gel bildet.

6. Mehrschichtige hämostatische Vorrichtung gemäß einem der vorhergehenden Ansprüche, wobei die dritte Schicht unter physiologischen Bedingungen nicht löslich und/oder nicht abbaubar ist und die zweite Schicht zwischen einer Wunde und der dritten Schicht angeordnet ist, wenn die hämostatische Vorrichtung auf die Wunde aufgebracht ist.

7. Mehrschichtige hämostatische Vorrichtung gemäß einem der vorhergehenden Ansprüche, wobei die oxidierte Cellulose oxidierte regenerierte Cellulose ist.

8. Mehrschichtige hämostatische Vorrichtung gemäß einem der Ansprüche 1 bis 7, wobei die dritte Schicht oxidierte regenerierte Cellulose umfasst und das hämostatische Mittel Fibrinogen umfasst, wobei das hämostatische Mittel vorzugsweise Fibrinogen ist.

9. Mehrschichtige hämostatische Vorrichtung gemäß einem der Ansprüche 1 bis 7, wobei die dritte Schicht oxidierte regenerierte Cellulose umfasst und das hämostatische Mittel Thrombin umfasst, wobei das hämostatische Mittel vorzugsweise Thrombin ist.

## Revendications

1. Un dispositif hémostatique multicouche comprenant :
Une première couche en contact avec la plaie comprenant de la carboxyméthylcellulose, la première couche étant soluble dans des conditions physiologiques, de sorte que la première couche se dissout lorsqu'elle est en contact avec un fluide provenant d'une plaie;
une deuxième couche comprenant un agent hémostatique en poudre fluide disposé sur la première couche, l'agent hémostatique comprenant l'un de fibrinogène, de thrombine, des monomères de fibrine ou la combinaison de thrombine et de gélatine; et
une troisième couche comprenant au moins l'un de gaze, de cellulose oxydée ou de collagène disposé sur la deuxième couche, dans lequel la première couche et la troisième couche encapsulent la deuxième couche,
dans lequel la première couche et la troisième couche ont des bords périmétriques et la première couche et la troisième couche sont scellées le long des bords périmétriques; et dans lequel le taux de dissolution de la première couche dans des conditions physiologiques est supérieur au taux de dégradation de la troisième couche dans des conditions physiologiques.

2. Le dispositif hémostatique multicouche selon la revendication 1, dans lequel l'agent hémostatique en poudre est une particule solide.

3. Le dispositif hémostatique multicouche selon la revendication 1 ou la revendication 2, dans lequel l'agent hémostatique en poudre est disposé sur la première couche selon un motif répétitif.

4. Le dispositif hémostatique multicouche selon la revendication 1, dans lequel les bords périmétriques de la première couche et de la troisième couche sont thermosoudés.

5. Dispositif hémostatique multicouche selon l'une des revendications précédentes, dans lequel la troisième couche forme un gel dans des conditions physiologiques.

6. Dispositif hémostatique multicouche selon l'une des revendications précédentes, dans lequel la troisième couche est insoluble et/ou non dégradable dans des conditions physiologiques et la deuxième couche est disposée entre une plaie et la troisième couche lorsque le dispositif hémostatique est appliqué sur la plaie.

7. Dispositif hémostatique multicouche selon l'une des revendications précédentes, dans lequel la cellulose oxydée est de la cellulose régénérée oxydée.

8. Dispositif hémostatique multicouche selon l'une des revendications 1 à 7, dans lequel la troisième couche comprend de la cellulose régénérée oxydée, et l'agent hémostatique comprend du fibrinogène, de préférence dans lequel l'agent hémostatique est du fibrinogène.

9. Dispositif hémostatique multicouche selon l'une des revendications 1 à 7, dans lequel la troisième couche comprend de la cellulose régénérée oxydée, et l'agent hémostatique comprend de la thrombine, de préférence dans lequel l'agent hémostatique est de la thrombine.
